# EUROPÄISCHE PATENTSCHRIFT

(11) **EP 2 125 201 B1**
(45) Veröffentlichungstag und Bekanntmachung des Hinweises auf die Patenterteilung: **13.04.2011**
(21) Anmeldenummer: 08716869.6
(22) Anmeldetag: 15.02.2008
(51) Int. Cl.: B01J 23/46, C07C 1/04, C07C 1/12, H01M 8/06

(54) **VERFAHREN ZUR SELEKTIVEN METHANISIERUNG VON KOHLENMONOXID**
METHOD FOR THE SELECTIVE METHANATION OF CARBON MONOXIDE
PROCÉDÉ DE MÉTHANISATION SÉLECTIVE DE MONOXYDE DE CARBONE

(30) Priorität: 23.02.2007 EP 07102945
(43) Veröffentlichungstag der Anmeldung: 02.12.2009
(73) Patentinhaber: BASF SE, 67056 Ludwigshafen (DE)
(72) Erfinder: STEINER, Jochen, 68165 Mannheim (DE); HÖLZLE, Markus, 67281 Kirchheim (DE); URTEL, Heiko, 68165 Mannheim (DE)
(86) Internationale Anmeldenummer: PCT/EP2008/051862
(87) Internationale Veröffentlichungsnummer: WO 2008/101875

(56) Entgegenhaltungen:
- EP-A- 1 712 278
- WO-A-2007/043442
- DE-A1- 3 737 419
- US-A1- 2002 115 563
- US-A1- 2004 175 310
- R.A.DALLA BETTA ET AL.: "Heterogeneous Methanation: Initial Rate of CO Hydrogenation on Supported Ruthenium and Nickel" JOURNAL OF CATALYSIS, Bd. 35, Nr. 1, 1974, Seiten 54-60, XP002482900

## Beschreibung

Die Erfindung betrifft die Verwendung einer katalytischen Zusammensetzung zur selektiven Methanisierung von Kohlenmonoxid in wasserstoff- und kohlendioxidhaltigen Strömen, insbesondere für die Verwendung in Brennstoffzellensystemen.

Niedertemperatur-PEM-Brennstoffzellen (PEM = Polymer Electrolyte Membrane) können nur mit Wasserstoff oder wasserstoffreichen Gasen einer definierten Qualität betrieben werden. Dabei ist insbesondere die Kohlenmonoxid (CO)-Konzentration eine kritische Größe. Sie hängt vom eingesetzten Energieträger und vom verwendeten Reformierungsverfahren ab. Die Entfernung von höheren CO-Konzentrationen ist mit der Wassergas-Shift-Reaktion unter weiterer Bildung von Wasserstoff möglich.

CO+H₂O ↔ CO₂+H₂ AH = -44 kJ/mol

Da es sich um eine Gleichgewichtsreaktion handelt, verbleibt in Abhängigkeit von Verfahrensauslegung und Temperatur eine Restkonzentration an CO im Gasstrom, in der Regel im Bereich von 0,25 bis 1,5 Vol.-%. Bei Verwendung von hoch kupferhaltigen Katalysatoren kann beispielsweise eine CO-Entfemung bis auf 2.500 ppm erreicht werden. Der CO-Gehalt im wasserstoffreichen Gas muss allerdings noch weiter reduziert werden, um eine Vergiftung des Anodenkatalysators zu vermeiden; Richtwerte liegen hier zwischen maximal 10 und 50 ppm.

Die Reduzierung des enthaltenen CO aus dem Gasstrom bis unter die erforderlichen Grenzwerte erfolgt üblicherweise in einer Feinreinigungsstufe. Dabei ist heute die selektive Oxidation die gängige CO-Entfernungsmethode. Die selektive Oxidation weist einen hohen Entwicklungsstand auf, besitzt aber neben dem Nachteil einer nur mäßigen Selektivität die Notwendigkeit einer exakt dosierten Luftzufuhr, woraus ein hoher mess- und regelungstechnischer Aufwand resultiert. Wird das notwendige Verhältnis von Sauerstoff zu CO nicht exakt eingehalten, kann dies zu hohen Verlusten an Wasserstoff führen. Weiterhin erfordert das schmale Temperaturfenster von in der Regel maximal 20°C ein aufwendiges Thermomanagement des Reaktors. Hinzu kommt über die Zumischung des Oxidationsmittels Sauerstoff zum Gas eine sicherheitstechnische Problematik. Die Entfernung des CO durch Reaktion mit H₂ (Methanisierung) hat gegenüber der selektiven CO-Oxidation durch ihre verfahrenstechnisch anspruchslose Realisierung erhebliche Vorteile.

Die CO-Methanisierung (Hydrierung von Kohlenstoffmonoxid zu Methan) erfolgt nach der Reaktionsgleichung:

CO + 3H₂ → CH₄ + H₂O AH = -206,2 kJ/mol

Als Konkurrenzreaktion läuft die Umwandlung von CO₂ zu Methan ab:

CO₂ + 4H₂ → CH₄ + 2H₂O ΔH = -164,9 kJ/mol

Die besondere Herausforderung für die selektive CO-Methanisierung liegt darin, dass bevorzugt CO hydriert werden soll und nicht CO₂, da dies weiteren Wasserstoff verbrauchen würde. Nach der Thermodynamik wird die CO-Methanisierung gegenüber der CO₂Methanisierung bevorzugt. Es ist bekannt, dass unterhalb eines Grenzwertes von 200 bis 300 ppm CO-Konzentration im Reformat die CO₂Methanisierung nicht einsetzt. Die CO-Konzentration liegt im Reformat bei ca. 15.000 ppm, also um den Faktor 50 höher als die angegebene Obergrenze. Der CO₂-Gehalt liegt mit ca. 15 bis 25 Vol.-% eine Größenordnung über dem CO-Gehalt. Dementsprechend ist ein COselektiver Katalysator bei niedrigen CO-Konzentrationen, wie sie für z. B. PEM-Brennstoffzellen verlangt werden, unabdingbar.

Die selektive Methanisierung von CO ist seit langem bekannt. Zunächst wurde CO am Nickelkatalysator methanisiert, wobei CO₂ jedoch zuvor ausgewaschen werden musste. 1968 wurde ein Rutheniumkatalysator zur selektiven CO-Methanisierung von Baker et al. beansprucht (US-A-3615164), wobei dort ein Ruthenium- oder Rhodiumkatalysator auf einem Aluminiumoxid-Trägermaterial verwendet wird. Ebenso ist in Chemical Abstracts, Band 74, 1971, Nr. 35106u, die selektive Methanisierung von CO in einem Gasgemisch, das Wasserstoff, Kohlendioxid und Kohlenmonoxid enthält, bei Temperaturen im Bereich zwischen 125 und 300°C unter Verwendung rutheniumhaltiger Katalysatoren beschrieben. In US-A-3663162 von 1972 wird ein Raney-Nickel-Katalysator für diese Reaktion beansprucht.

US-A-2006/0111456 beschreibt die selektive Methanisierung von CO in wasserstoffreichen Strömen unter Einsatz eines platin- und ruthenium-haltigen Katalysators auf einem Metallträger, der Aluminiumoxid, Cerdioxid, Zirkonoxid oder Mischungen davon enthält.

US-A-3787468 beschreibt die Methanisierung von CO und/oder CO₂ in Gegenwart eines rhodium- oder rutheniumhaltigen Katalysators, der Wolframoxid und bevorzugt auch Platin enthält.

In EP-A-1174486 wird eine Methanisierungsstufe mit einer Einheit zur selektiven Oxidation mit dem Ziel eines geringeren Sauerstoffverbrauches und einer geringeren CO₂-Methanisierungsrate kombiniert. Der für die Methanisierung eingesetzte Katalysator enthält Ru, Pt, Rh, Pd oder Ni auf einem Aluminiumoxidträger.

In EP-A-0946406 werden zwei Methanisierungsstufen unterschiedlicher Temperaturniveaus zusammengeschaltet. Vorteil soll hier sein, dass bei der Hochtemperaturstufe noch kein oder weniger CO₂ methanisiert, aber schon ein großer Teil des Kohlenmonoxids abgebaut wird. In der sich anschließenden Tieftemperaturmethanisierung erfolgt die Restentfemung von CO. Verwendet wird ein Edelmetallkatalysator, insbesondere Rh, auf einem Aluminiumträger.

WO 97/43207 beschreibt die Kombination einer ersten Stufe zur selektiven Oxidation mit einer nachfolgenden Methanisierungsstufe mit Rhodium als Aktivkomponente. Mit dieser Kombination sollen sich beide Prozesse unter optimalen Bedingungen betreiben lassen.

Weitere neuere Anmeldungen, wie beispielsweise EP-A-1246286, in der als letzte Prozessstufe einer Gasreinigung ein Methanisierungsreaktor einer Einheit zur selektiven Oxidation mit der Begründung des einfacheren Aufbaus und der besseren Handhabung nachgeschaltet wird, verwenden herkömmliche Katalysatoren, überwiegend auf Ruthenium- oder Nickelbasis.

JP-A-2002/068707 behandelt Methanisierungskatalysatoren, die auf einem feuerfesten anorganischen Oxid, ausgewählt aus Oxiden von Aluminium, Titan, Silizium oder Zirkonium, aufgebracht sind.

EP-A-1707261 beschreibt ein Verfahren zur selektiven Oxidation von CO mit Ru auf einem Katalysator aus Mischmetalloxiden, dotiert mit Lanthaniden.

US-A-2005/0096212 beschreibt die selektive Methanisierung an einem Katalysator aus Ru, Rh, Ni oder Kombinationen auf β-Zeolith, Mordenit und Faujasit. Zwar werden so die gewünschten CO-Konzentrationen unterhalb von 100 ppm erreicht, doch sinkt die Selektivität bei Temperaturen über 190°C, bei denen der Katalysator seine Aktivität entfaltet, deutlich unter 50%. Da die Hydrierung von CO₂ pro Mol 3/2 so viel Wasserstoff vernichtet wie die Hydrierung von CO, ist die Forderung nach möglichst hoher Selektivität sehr wichtig. Außerdem wird eine vernünftige katalytische Aktivität nur über das sehr kleine Temperaturfenster zwischen 170°C und 180°C erreicht.

Die Verfahren des Standes der Technik gestatten es nicht, eine ausreichende Senkung des CO-Gehaltes unter Schonung des CO₂-Gehaltes zu gewährleisten. Die bisher entwickelten Katalysatoren arbeiten entweder nicht selektiv genug bzw. wirken nur in einem sehr schmalen Temperaturbereich. Vor allem der sehr schmale Temperaturbereich macht eine technische Realisierung des Konzeptes "Selektive Methanisierung" sehr schwierig. Denn sobald die Selektivität sinkt, kommt es zur Erwärmung des Reaktors, was zu einer weiteren Methanisierung von CO₂ und damit zum thermischen "Durchgehen" der Prozesseinheit führt. Durch die Exothermie der Reaktion kommt es also zu Hot-Spots. Aus diesem Grund muss ein breites Temperaturfenster fahrbar sein. Ebenso ein Problem ist die adiabate Temperaturerhöhung in Monolithen, wenn diese als Katalysatorformkörper eingesetzt werden, was in der Praxis oftmals der Fall ist.

Insbesondere für Brennstoffzellenanwendungen stellt der geforderte CO-Maximalgehalt im eingespeisten wasserstoffreichen Gas und die notwendige hohe Selektivität (Methanisierung von CO, aber nicht von CO₂) über ein breites Temperaturfenster noch ein großes Entwicklungspotential für geeignete deaktivierungsresistente Katalysatoren dar.

Die Aufgabe der Erfindung bestand damit in der Bereitstellung eines Katalysators für die selektive CO-Methanisierung, der seine Selektivität und Aktivität in einem breiten Temperaturbereich erhält.

Die Aufgabe wurde erfindungsgemäß dadurch gelöst, dass für die selektive Methanisierung von Kohlenmonoxid in wasserstoff- und kohlendioxidhaltigen Strömen eine katalytisch aktive Zusammensetzung eingesetzt wird, die als Aktivkomponente Ruthenium und als Trägermaterial ein Lanthan-Cer-Zirkönoxid (LaCeZr-Oxid) enthält.

Ein Katalysator, der als Trägermaterial ein Lanthan-Cer-Zirkonoxid enthält und Ruhenium als Aktivkomponente trägt, ist in der Lage, die Methanisierung von CO in einem breiten Temperaturbereich in einer nahezu konstanten Selektivität über eine lange Zeitspanne zu gewährleisten. Herkömmliche Katalysatoren zeigen mit zunehmender Temperatur und längeren Laufzeiten einen deutlichen Selektivitätsabfall. Durch Anwendung des erfindungsgemäßen Katalysators ist ein deutlich geringerer Regelaufwand erforderlich, da das Temperaturfenster bei der Methanisierung des CO weniger exakt eingehalten werden muss. Darüber hinaus kann ein auch bei hohen Temperaturen gut arbeitender Katalysator direkt der Vorreinigungsstufe (TTK - Tieftemperaturkonvertierung), die bei etwa 220 bis 280°C betrieben wird, nachgeschaltet werden.

Gegenstand der Erfindung ist damit die Verwendung einer katalytisch aktiven Zusammensetzung für die selektive Methanisierung von Kohlenmonoxid in wasserstoff- und kohlendioxidhaltigen Strömen, die dadurch gekennzeichnet ist, dass sie als Aktivkomponente Ruthenium und als Trägermaterial ein Lanthan-Cer-Zirkonoxid enthält, wobei die Gesamtbeladung des Trägermaterials mit der Aktivkomponente 0,1 bis 20 Gew.-%, bezogen auf das Gesamtgewicht der katalytisch aktiven Zusammensetzung, beträgt und das Trägermaterial einen Lanthanoxid-Gehalt von 0,1 bis 15 Gew.-%, einen Ceroxid-Gehalt von 0,1 bis 15 Gew.-% und einen Zirkonoxid-Gehalt von 30 bis 99,8 Gew.-%, bezogen auf das Gewicht des gesamten Trägermaterials, enthält.

Die Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in den jeweils angegebenen Kombinationen, sondern auch in anderen Kombinationen verwendbar sind, ohne den Rahmen der Erfindung zu verlassen.

Als Trägermaterial wird erfindungsgemäß ein Lanthan-Cer-Zirkonoxid (LaCeZr-Oxid) eingesetzt.

Das Trägermaterial weist vorteilhafterweise einen Lanthanoxid-Gehalt von 0,1 bis 15 Gew.-%, bevorzugt von 5 bis 15 Gew.-% und besonders bevorzugt von 10 bis 15 Gew.-%, auf. Der Ceroxid-Gehalt beträgt vorteilhafterweise 0,1 bis 15 Gew.-%, bevorzugt 0,1 bis 10 Gew.-% und besonders bevorzugt von 3 bis 7 Gew.-%, jeweils bezogen auf das Gewicht des gesamten Trägermaterials.

Der Zirkonoxid-Gehalt des Trägermaterials beträgt vorteilhafterweise 30 bis 99,8 Gew.-%. In bevorzugten Ausführungen liegt er bei einem Gehalt, der die Gewichtsanteile des Lanthanoxids und Ceroxids und ggf. weiterer Bestandteile, wie nachfolgend beschrieben, zu jeweils 100 Gew.-% ergänzt.

Das erfindungsgemäß verwendete Trägermaterial kann neben den genannten Komponenten weitere üblicherweise in der Katalysatorchemie für diese Zwecke einsetzbare Materialien enthalten, wie beispielsweise Aluminiumoxid. Geeignet sind solche Bindermaterialien, die eine ausreichend hohe BET-Oberfläche aufweisen. Vorteilhafterweise sollte die BET-Oberfläche dieser zusätzlich eingesetzten Bindermaterialien mindestens 120 m²/g betragen. Der Gehalt an diesen weiteren Materialien sollte dabei 30 Gew.-%, vorzugsweise 20 Gew.-%, jeweils bezogen auf das Gewicht des gesamten Trägermaterials, nicht überschreiten.

Die katalytisch aktive Zusammensetzung enthält als Aktivkomponente Ruthenium. Die Aktivkomponente liegt dabei im Katalysator vorzugsweise als Oxid vor. Die eigentliche Aktivmasse wird dann durch Aktivierung mit Wasserstoff in situ erzeugt.

Die Beladung des Trägermaterials mit der Aktivkomponente Ruthenium beträgt vorteilhafterweise 0,1 bis 20 Gew.-%, vorzugsweise 0,1 bis 10 Gew.-% und besonders bevorzugt 0,1 bis 5 Gew.-%. Weitere vorteilhafte Mengenbereiche sind beispielsweise 1 bis 10 Gew.-%, 1 bis 5 Gew.-% sowie 2 bis 5 und 3 bis 5 Gew.-%. Die Angaben sind jeweils bezogen auf das Gesamtgewicht der katalytisch aktiven Zusammensetzung.

Eine bevorzugte Zusammensetzung des katalytisch aktiven Systems enthält auf einem Lanthan-Cer-Zirkonoxid-Träger mit einem Lanthanoxid-Gehalt von 0,1 bis 15 Gew.-% und einem Ceroxid-Gehalt von 0,1 bis 15 Gew.-%, jeweils bezogen auf das Gewicht des gesamten Trägermaterials, 0,1 bis 20 Gew.-% Ru, bezogen auf das Gesamtgewicht der katalytisch aktiven Zusammensetzung.

Eine weitere bevorzugte Zusammensetzung des katalytisch aktiven Systems enthält auf einem Lanthan-Cer-Zirkonoxid-Träger mit einem Lanthanoxid-Gehalt von 0,1 bis 15 Gew.-% und einem Ceroxid-Gehalt von 0,1 bis 15 Gew.-%, jeweils bezogen auf das Gewicht des gesamten Trägermaterials, 2 bis 5 Gew.-% Ru, bezogen auf das Gesamtgewicht der katalytisch aktiven Zusammensetzung.

Eine weitere bevorzugte Zusammensetzung des katalytisch aktiven Systems enthält auf einem Lanthan-Cer-Zirkonoxid-Träger mit einem Lanthanoxid-Gehalt von 0,1 bis 15 Gew.-% und einem Ceroxid-Gehalt von 0,1 bis 10 Gew.%, jeweils bezogen auf das Gewicht des gesamten Trägermaterials, 3 bis 5 Gew.-% Ru, bezogen auf das Gesamtgewicht der katalytisch aktiven Zusammensetzung.

Eine besonders bevorzugte Zusammensetzung des katalytisch aktiven Systems enthält auf einem Lanthan-Cer-Zirkonoxid-Träger mit einem Lanthanoxid-Gehalt von 10 bis 15 Gew.-% und einem Ceroxid-Gehalt von 3 bis 7 Gew.-%, jeweils bezogen auf das Gewicht des gesamten Trägermaterials, 3 bis 5 Gew.-% Ru, bezogen auf das Gesamtgewicht der katalytisch aktiven Zusammensetzung.

Weitere Ausführungsformen in der Zusammensetzung des erfindungsgemäß eingesetzten Katalysators sind den Beispielen zu entnehmen. Es versteht sich, dass die vorstehend genannten und nachstehend noch angegebenen Merkmale des Katalysators nicht nur in den angegebenen Kombinationen und Wertebereichen, sondern auch in anderen Kombinationen und Wertebereichen in den Grenzen des Hauptanspruchs verwendbar sind, ohne den Rahmen der Erfindung zu verlassen.

Des Weiteren können die Aktivkomponente und/oder das Trägermaterial mit für diese Zwecke einsetzbaren und dem Fachmann bekannten weiteren Elementen in geringen Mengen dotiert werden, ohne dass der Rahmen der Erfindung verlassen wird.

Die Herstellung des erfindungsgemäß eingesetzten Katalysators erfolgt auf übliche Art und Weise, beispielsweise indem die Aktivkomponente und ggf. Dotierelemente, vorzugsweise in Form ihrer Salze/Hydrate, in Lösung gebracht und dann in geeigneter Weise, beispielsweise durch Tränken, auf den Lanthan-Cer-Zirkonoxid-Träger aufgetragen werden. Danach wird der Katalysator getrocknet, kalziniert, ggf. reduziert und ggf. passiviert.

Das Aufbringen der Aktivkomponenten durch Tränken auf das Trägermaterial kann auf übliche Art und Weise erfolgen, wie z. B. als Washcoat auf einen Monolithen. Durchführung und Verfahrensbedingungen sind beispielsweise im Handbook of heterogeneous catalysis, Vol. 1, VCH Verlagsgesellschaft Weinheim, 1997, beschrieben.

Eine alternative Herstellungsweise beinhaltet die Verknetung der Trägermaterialien mit den Salzen/Hydraten der Aktiv- und ggf. Dotierelemente mit anschließender Verstrangung, Trocknung und ggf. Kalzination, ggf. Reduktion und ggf. Passivierung.

Dabei können das Verkneten des Trägermaterials mit den Aktivmassen sowie die weiteren Arbeitsschritte auf übliche Art und Weise mit bekannten Apparaturen erfolgen. Die Herstellung von Formkörpern aus pulverförmigen Rohstoffen kann durch übliche, dem Fachmann bekannte Methoden, wie beispielsweise Tablettierung, Aggregation oder Extrusion erfolgen, wie sie u.a. im Handbook of Heterogeneous Catalysis, Vol. 1, VCH Verlagsgesellschaft Weinheim, 1997, beschrieben sind, erfolgen.

Bei der Verformung bzw. dem Aufbringen können dem Fachmann bekannte Hilfsstoffe, wie Binder, Schmiermittel und/oder Lösungsmittel, zugesetzt werden.

Es entsteht eine katalytisch aktive Zusammensetzung, die für die selektive Methanisierung von Kohlenmonoxid in wasserstoff- und kohlendioxidhaltigen Strömen hervorragend geeignet ist. In Abhängigkeit von den jeweiligen Reaktionsbedingungen wird dabei die gewünschte deutliche Abreicherung des CO kleiner 10ppm im Gasgemisch unter minimalem Verlust an Wasserstoff erreicht.

Vorteilhafterweise wird damit die selektive Methanisierung des CO in einem Temperaturbereich von vorzugsweise 100 bis 300°C erreicht.

Besonders vorteilhaft ist die selektive Methanisierung von CO in einem Temperaturbereich von 180 bis 260°C. Diese Temperatur ermöglicht eine direkte thermische Integration an die vorgeschaltete Tieftemperaturkonvertierung. Es wird damit möglich, die erfindungsgemäße Methanisierungsstufe unmittelbar an die Tieftemperaturkonvertierungsstufe anzukoppeln. Durch die hohe Aktivität bei gleichermaßen hoher CO-Selektivität in diesem Temperaturbereich wird gewährleistet, dass ein stabiler und vor allem thermisch integrierter Betrieb des Katalysators erst möglich wird.

Die katalytisch aktive Zusammensetzung eignet sich damit hervorragend für CO-Feinreinigungen in wasserstoff- und kohlendioxidhaltigen Strömen, insbesondere für den Einsatz bei der Wasserstofferzeugung für Brennstoffzellenanwendungen.

Die Erfindung wird anhand der nachfolgenden Ausführungsbeispiele näher erläutert, ohne jedoch hierdurch eine entsprechende Eingrenzung vorzunehmen.

### Beispiele

Beispiel 1:

206,3 g ZrO₂, 29,6 g La₂(NO₃)₂, 0,24 g CeO₂ und 31,3 g Aluminiumoxid-Hydroxid (Pural SB) wurden im Kneter gemischt und mit verdünnter HNO₃ angesäuert. Es wurde so viel Wasser zugegeben, bis eine verstrangbare Masse entstand. Die ausgeformten Stränge wurden getrocknet und kalziniert. Dieser Träger wurde im Anschluss mit einer RuCl₃-Lösung versetzt, deren Konzentration so eingestellt war, dass das nochmals kalzinierte Endprodukt 3 Gew.-% Ru als Aktivmasse trug.

### Beispiel 2:

Aus 279,8 g ZrO₂, 12,5 g CeO₂, 31,5 g La₂(NO₃)₂ und 33,3 g Aluminiumoxid-Hydroxid (Pural SB) wurde ein Trägermaterial wie in Beispiel 1 beschrieben hergestellt. Dieser Träger wurde im Anschluss mit einer RuCl₃-Lösung versetzt, deren Konzentration so eingestellt war, dass das nochmals kalzinierte Endprodukt 3 Gew.-% Ru als Aktivmasse trug.

### Beispiel 3:

### Ein Träger aus 70 Gew.-% ZrO₂, 15 Gew.-% CeO₂, 5 Gew.-% La₂O₃ und 10 Gew.-% Al₂O₃ wurde mit einer RuCl₃-Lösung versetzt, deren Konzentration so eingestellt war, dass das nochmals kalzinierte Endprodukt 3 Gew.-% Ru als Aktivmasse trug.

### Beispiel 4: (Vergleichsbeispiel)

37,5 g PZ2-25 H (H-ZSM-5, MFI-Strukturtyp, Fa. Zeochem, Modul = 25) wurden mit 11,8 g Aluminiumoxid-Hydroxid (Versal 250, Fa. UOP) im Kneter vorgelegt und mit Ameisensäure leicht angeätzt. Die Masse wurde mit Wasser versetzt, verstrangt und kalziniert. Dieser Träger wurde im Anschluss mit einer Lösung aus Ru-Chlorid-Hydrat und La-Nitrat getränkt, die so eingestellt war, dass das Endprodukt 3 Gew.-% Ru und 5 Gew.-% La enthielt. Die Stränge wurden getrocknet und kalziniert.

### Beispiel 5: (Vergleichsbeispiel)

100 g PZ2-25 H (H-ZSM-5, MFI-Strukturtyp, Fa. Zeochem, Modul = 25) wurden mit 35,6 g Aluminiumoxid-Hydroxid (Pural SB) im Kneter vorgelegt und mit Ameisensäure leicht angeätzt. Die Masse wurde mit Wasser, Ru-Chlorid-Hydrat und Fe-Chlorid-Hydrat versetzt und verstrangt. Nach Kalzination enthielt das Endprodukt 3 Gew.-% Ru und 1 Gew.-% Fe.

### Beispiel 6: (Vergleichsbeispiel)

Testung eines kommerziell erhältlichen Methanisierkatalysator mit 5% Ru auf TiO₂.

### Beispiel 7a-d:

Ein Träger aus 70 Gew.-% ZrO₂, 15 Gew.-% CeO₂, 5 Gew.-% La₂O₃ und 10 Gew.-% Al₂O₃ wurde mit einer RuCl₃-Lösung versetzt, deren Konzentration so eingestellt war, dass das nochmals kalzinierte Endprodukt 5 Gew.-% Ru (Beispiel 7a), 4 Gew.-% Ru (Beispiel 7b), 3 Gew.-% Ru (Beispiel 7c) bzw. 2 Gew.-% Ru (Beispiel 7d) als Aktivmasse trug.

### Beispiel 8:

### (Patentnachstellung US 2005/096211)

150 g TZB 213 (β-Zeolith, Fa. Süd-Chemie/Tricat, Modul = 12) wurden mit 50 g Aluminiumoxid-Hydroxid (Pural SB) im Kneter vorgelegt und mit Ameisensäure leicht angeätzt. Die Masse wurde mit Wasser versetzt, verstrangt und kalziniert. Dieser Träger wurde im Anschluss mit einer Lösung aus Ru-Nitrosylnitrat getränkt, die so eingestellt war, dass das Endprodukt 3 Gew.-% Ru enthielt. Die Stränge wurden getrocknet und kalziniert.

### Beispiel 9:

### (Patentnachstellung JP 2002/068707)

432,8 g ZrO₂-Pulver wurden mit 12 g Methylcellulose (Walocel, Fa. Wolff Cellulosics) im Kneter vorgelegt und mit Salpetersäure leicht angeätzt. Die Masse wurde mit Wasser und Ru-Chlorid-Hydratlösung versetzt und verstrangt. Nach Kalzination enthielt das Endprodukt 3 Gew.-% Ru.

### Beispiel 10:

### (Patentnachstellung JP 2002/068707)

250 g Aluminiumoxid-Hydroxid (Pural SB) wurden im Kneter vorgelegt und mit Ameisensäure leicht angeätzt. Die Masse wurde mit Wasser und Ru-Chlorid-Hydratlösung versetzt und verstrangt. Nach Kalzination enthielt das Endprodukt 3 Gew.-% Ru.

### Beispiel 11:

### (Patentnachstellung JP 2002/068707)

235,5 g TiO₂-Pulver wurden im Kneter vorgelegt und mit Ameisensäure leicht angeätzt. Die Masse wurde mit Wasser und Ru-Chlorid-Hydratlösung versetzt und verstrangt. Nach Kalzination enthielt das Endprodukt 3 Gew.-% Ru.

### Testbedingungen:

Für den Versuch wurde ein elektrisch beheizter Rohrreaktor mit einem Volumen von 50 ml und einem Durchmesser von 14 mm verwendet.

Zuunterst wurden 4 ml Steatit-Kugeln mit einem Durchmesser von 1,8 bis 2,2 mm eingebaut, auf die anschließend die Katalysatormischung gegeben wurde. Die Katalysatormischung bestand aus ca. 20 ml Katalysator, der mit ca. 10 ml Steatit-Kugeln mit einem Durchmesser von 1,8 bis 2,2 mm gut durchmischt wurde. Als Vorschüttung dienten 14 ml Steatit-Kugeln mit einem Durchmesser von 1,8 bis 2,2 mm, die das Restvolumen des Reaktors füllten.

Der Katalysator wurde zunächst mit 90 l/h Stickstoff und 10 l/h Wasserstoff bei 230°C eine Stunde lang reduziert. Die für den Versuch gewählte Gaszusammensetzung ist typisch für den Ausgang der Tieftemperatur-Shiftstufe nach der Reformierung von Methan und betrug 33 Vol.-% H₂, 28 Vol.-% N₂, 25 Vol.-% H₂O, 13 Vol.-% CO₂, 0,5 Vol.-% CO und 0,5 Vol.-% CH₄. Es wurde eine Belastung von 5000 l·h⁻¹·l⁻¹_{Kat} gewählt. Nachdem alle Gase eingestellt und der Reaktor (nach der Reduktion bei 230°C) auf 150°C abgekühlt war, wurde der Versuch gestartet. Alle drei Stunden wurde die Temperatur schrittweise erhöht, die Maximaltemperatur betrug 300°C. Die Konzentration der Gase wurde mittels GC und IR hinter dem Reaktor bestimmt.

Unter den genannten Bedingungen wurden die Katalysatoren vermessen.

Eine Selektivität größer als 60 % wurde als zufriedenstellend erachtet. Die Selektivität sinkt mit steigender Temperatur. In der folgenden Tabelle 1 sind jeweils die Temperaturen angegeben, bei denen die Selektivität diese Größe unterschreitet bzw. die Temperatur, ab der CO auf unter 10 ppm abgereichert wird. Die letzte Spalte gibt die Größe des Temperaturfensters an, bei dem sowohl ausreichende Aktivität (die zu weniger als 10 ppm CO hinter dem Reaktor führt) als auch ausreichende Selektivität (> 60 %) realisiert werden.

**Tab. 1: Auflistung ausgewählter und getesteter Kontakte**

| Katalysator | | Temperatur [°C], ab der die | | Temperatur- |
|---|---|---|---|---|
| - | Aktivmasse | Selektivität | Aktivität | bereich [K], |
| - | Träger | < 60% | CO < 10 ppm | mit CO <10 |
| | | | | ppm und |
| | | | | Selektivität |
| | | | | > 60% |
| Beispiel 1: | 3% Ru | > 260 | 210 | > 50 |
| | LaCeZr-Oxid (Typ I) | | | |
| Beispiel 2: | 3% Ru | > 260 | 210 | > 50 |
| | LaCeZr-Oxid (Typ II) | | | |
| Beispiel 3: | 3% Ru | 265 | 200 | 65 |
| | LaCeZr-Oxid (Typ III) | | | |
| Beispiel 4: | 3% Ru und 5% La | > 260 | -* | 0 |
| | ZSM-5 Zeolith | | | |
| Beispiel 5: | 3% Ru und 1 % Fe | 245 | 225 | 20 |
| | ZSM-5 Zeolith | | | |
| Beispiel 6: | 5% Ru | 225 | -* | 0 |
| | TiO₂ | | | |
| Beispiel 7a: | 5% Ru | 245 | 175 | 70, |
| | LaCeZr-Oxid (Typ III) | | | |
| Beispiel 7b: | 4% Ru | 245 | 190 | 55 |
| | LaCeZr-Oxid (Typ III) | | | |
| Beispiel 7c: | 3% Ru | 265 | 200 | 65 |
| | LaCeZr-Oxid (Typ III) | | | |
| Beispiel 7d: | 2% Ru | > 280 | 220 | > 60 |
| | LaCeZr-Oxid (Typ III) | | | |
| Beispiel 8 | 3% Ru | > 260 | 220 | 40 |
| | β -Zeolith | | | |
| Beispiel 9 | 3%Ru | 200 | 200 | 0 |
| | ZrO₂ | | | |
| Beispiel 10 | 3% Ru | > 260 | -* | 0 |
| | Al₂O₃ | | | |
| Beispiel 11 | 3% Ru | - | -* | 0 |
| | TiO₂ | | | |

| | | | | |
|---|---|---|---|---|
| *: Zielwert von 10 ppm wird nicht erreicht | | | | |

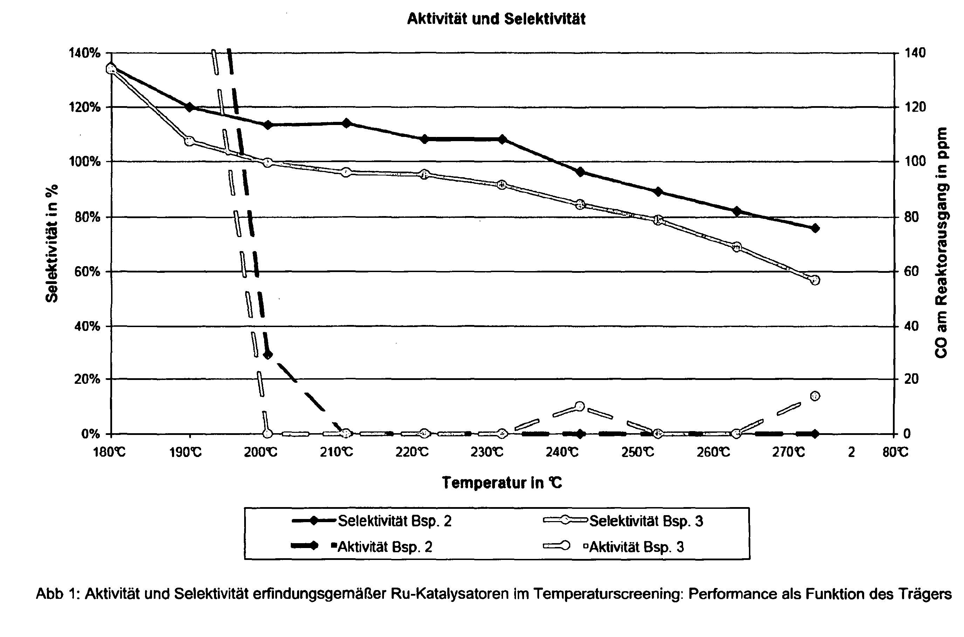

Abbildung 1 zeigt die Aktivität und Selektivität ausgewählter Ru-Katalysatoren im Temperaturscreening (Performance als Funktion unterschiedlicher LaCeZrOx Träger bei gleichem Ru-Gehalt). Man erkennt den breiten Temperaturbereich in dem die erfindungsgemäß eingesetzten Katalysatoren vollen CO-Umsatz unter Einhaltung der notwendig hohen Selektivität zeigen.

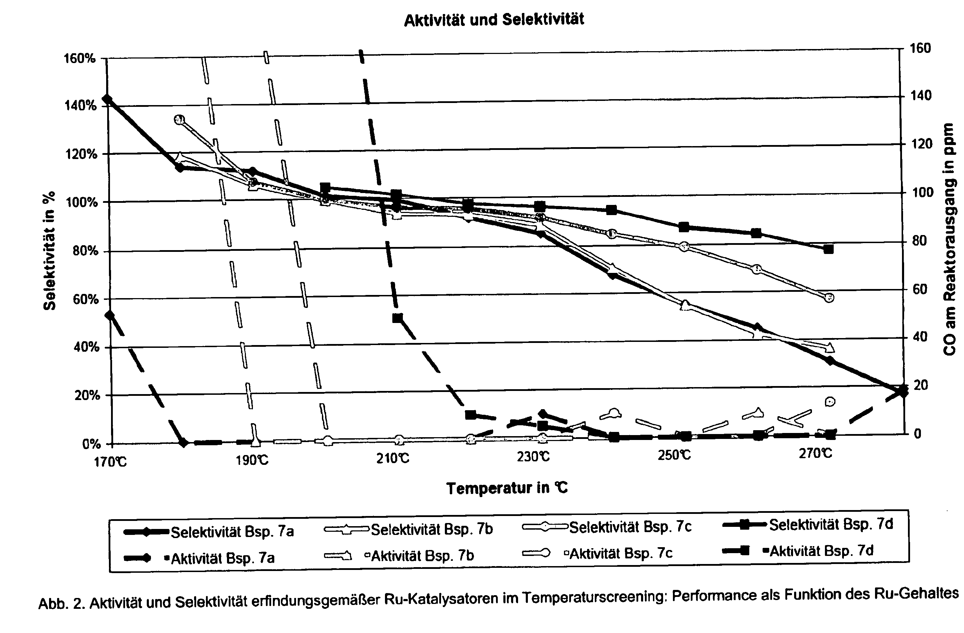

Abbildung 2 zeigt die Aktivität und Selektivität ausgewählter Ru-Katalysatoren im Temperaturscreening (Performance als Funktion des Ru-Gehaltes). Man erkennt den Einfluss der Ru-Konzentration auf den Aktivitätsbereich der einzelnen Katalysatoren unter Einhaltung einer hohen Selektivität.

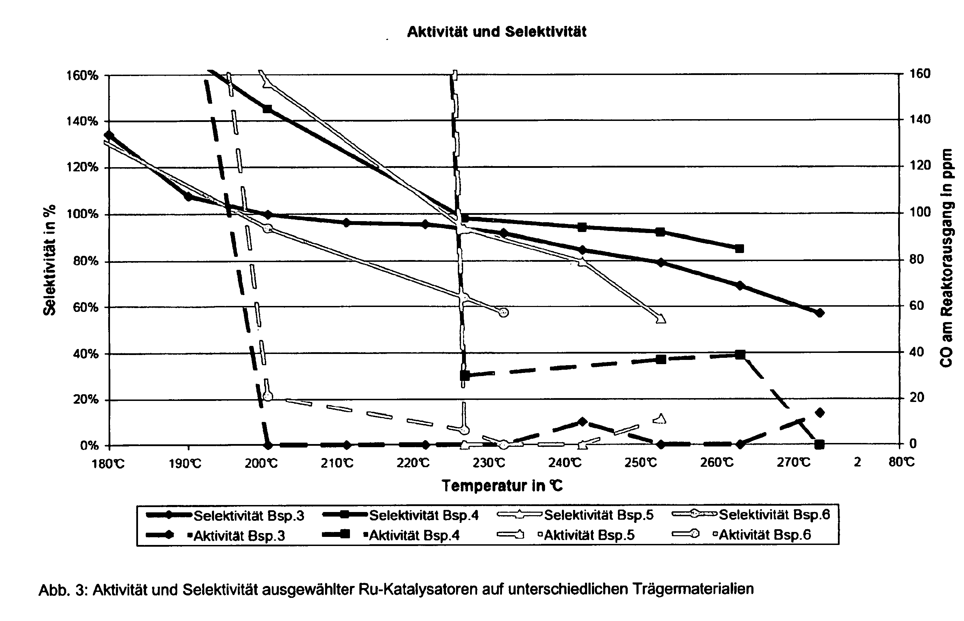

Abbildung 3 zeigt die Aktivität und Selektivität ausgewählter Ru-Katalysatoren auf unterschiedlichen Trägermaterialien. Damit wird die Überlegenheit des erfindungsgemäß eingesetzten Katalysators auf Basis von Lanthan-Cer-Zirkonoxid als Trägermaterial gegenüber Katalysatoren des Standes der Technik demonstriert.

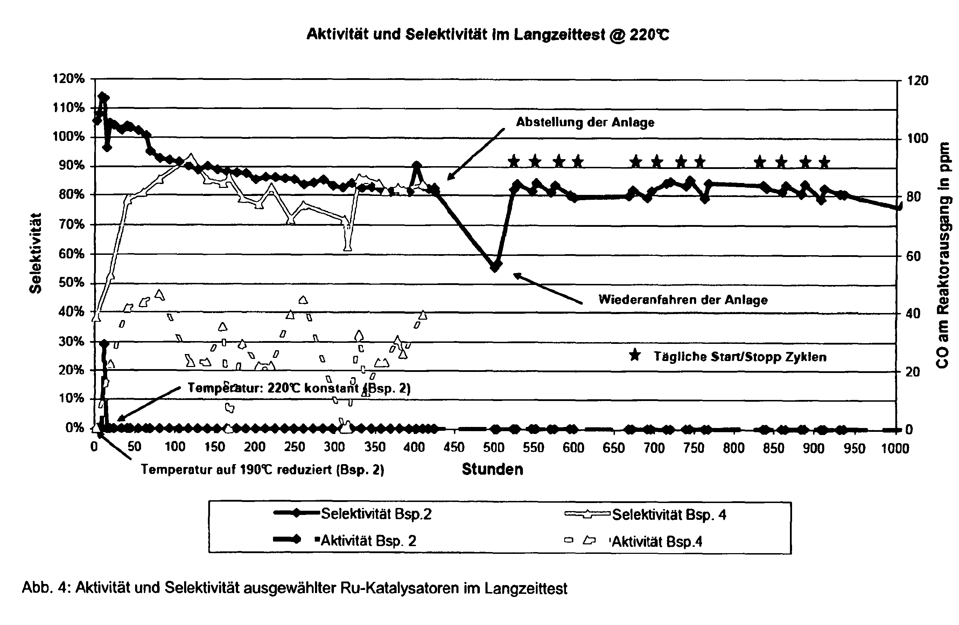

Abbildung 4 zeigt Aktivität und Selektivität ausgewählter Ru-Katalysatoren im Langzeittest.

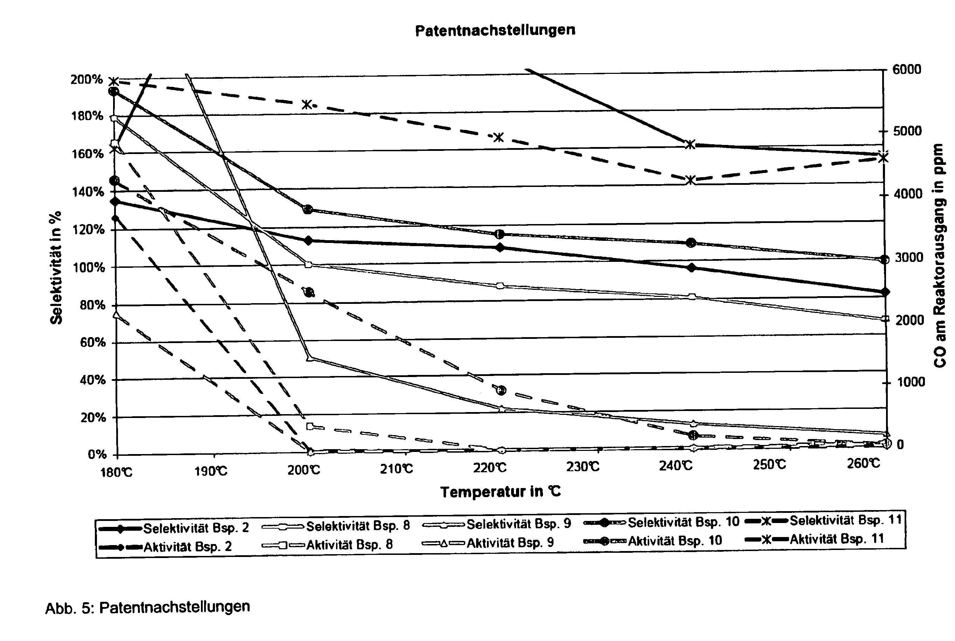

Abbildung 5 belegt die deutlich höhere Aktivität und Selektivität des erfindungsgemäß eingesetzten Katalysators im Vergleich zum Stand der Technik und bestehenden Patentanmeldungen.

## Patentansprüche

1. Verwendung einer katalytisch aktiven Zusammensetzung enthaltend als Aktivkomponente Ruthenium und als Trägermaterial ein Lanthan-Cer-Zirkonoxid für die selektive Methanisierung von Kohlenmonoxid in wasserstoff und kohlendioxidhaltigen Strömen, wobei die Gesamtbeladung des Trägermaterials mit der Aktivkomponente 0,1 bis 20 Gew.-%, bezogen auf das Gesamtgewicht der katalytisch aktiven Zusammensetzung, beträgt und das Trägermaterial einen Lanthanoxid-Gehalt von 0,1 bis 15 Gew.-%, einen Ceroxid-Gehalt von 0,1 bis 15 Ges.-% und einen Zirkonoxid-Gehalt von 30 bis 99,8 Gew.-%, bezogen auf das Gewicht des gesamten Trägermaterials, enthält.

2. Verwendung einer katalytisch aktive Zusammensetzung gemäß Anspruch 1, **dadurch gekennzeichnet, dass** die Gesamtbeladung des Trägermaterials mit der Aktivkomponente 0,1 bis 10 Gew.-%, bezogen auf das Gesamtgewicht der katalytisch aktiven Zusammensetzung, beträgt.

3. Verwendung einer katalytisch aktive Zusammensetzung gemäß Anspruch 1 oder 2, **dadurch gekennzeichnet, dass** die Gesamtbeladung des Trägermaterials mit der Aktivkomponente 2 bis 5 Gew.-%, bezogen auf das Gesamtgewicht der katalytisch aktiven Zusammensetzung, beträgt.

4. Verwendung einer katalytisch aktive Zusammensetzung gemäß einem der Ansprüche 1 bis 3, **dadurch gekennzeichnet, dass** die katalytisch aktive Zusammensetzung einen Lanthan-Cer-Zirkonoxid-Träger mit einem Lanthanoxid-Gehalt von 10 bis 15 Gew.-% und einem Ceroxid-Gehalt von 3 bis 7 Gew.-%, jeweils bezogen auf das Gewicht des gesamten Trägermaterials, sowie 2 bis 5 Gew.-% Ru, bezogen auf das Gesamtgewicht der katalytisch aktiven Zusammensetzung, enthält.

## Claims

1. The use of a catalytically active composition comprising ruthenium as an active component and a lanthanum-cerium-zirconium oxide as a support material for the selective methanization of carbon monoxide in hydrogen and carbon dioxide-containing streams, where the total loading of the support material with the active component is 0.1 to 20% by weight, based on the total weight of the catalytically active composition, and the support material comprises a lanthanum oxide content of 0.1 to 15% by weight, a cerium oxide content of 0.1 to 15% by weight and a zirconium oxide content of 30 to 99.8% by weight, based on the weight of the overall support material.

2. The use of a catalytically active composition according to claim 1, wherein the total loading of the support material with the active component is 0.1 to 10% by weight, based on the total weight of the catalytically active composition.

3. The use of a catalytically active composition according to claim 1 or 2, wherein the total loading of the support material with the active component is 2 to 5% by weight, based on the total weight of the catalytically active composition.

4. The use of a catalytically active composition according to any one of claims 1 to 3, wherein the catalytically active composition comprises a lanthanum-cerium-zirconium oxide support with a lanthanum oxide content of 10 to 15% by weight and a cerium oxide content of 3 to 7% by weight, based in each case on the weight of the overall support material, and 2 to 5% by weight of Ru, based on the total weight of the catalytically active composition.

## Revendications

1. Utilisation d'une composition catalytiquement active contenant comme composant actif du ruthénium et comme matériau support un oxyde de lanthane-cérium-zirconium pour la méthanisation sélective de monoxyde de carbone dans des flux contenant de l'hydrogène et du dioxyde de carbone, où la charge totale du matériau support par le composant actif est de 0,1 à 20% en poids, par rapport au poids total de la composition catalytiquement active et le matériau support contient une teneur en oxyde de lanthane de 0,1 à 15% en poids, une teneur en oxyde de cérium de 0,1 à 15% en poids et une teneur en oxyde de zirconium de 30 à 99,8% en poids, par rapport au poids de la totalité du matériau support.

2. Utilisation d'une composition catalytiquement active selon la revendication 1, **caractérisée en ce que** la charge totale du matériau support par le composant actif est de 0,1 à 10% en poids, par rapport au poids total de la composition catalytiquement active.

3. Utilisation d'une composition catalytiquement active selon la revendication 1 ou 2, **caractérisée en ce que** la charge totale du matériau support par le composant actif est de 2 à 5% en poids, par rapport au poids total de la composition catalytiquement active.

4. Utilisation d'une composition catalytiquement active selon l'une quelconque des revendications 1 à 3, **caractérisée en ce que** la composition catalytiquement active contient un support en oxyde de lanthane-cérium-zirconium présentant une teneur en oxyde de lanthane de 10 à 15% en poids et une teneur en oxyde de cérium de 3 à 7% en poids, à chaque fois par rapport au poids de la totalité du matériau support, ainsi que 2 à 5% en poids de Ru, par rapport au poids total de la composition catalytiquement active.
